# EUROPÄISCHE PATENTSCHRIFT

(11) **EP 1 868 543 B1**
(45) Veröffentlichungstag und Bekanntmachung des Hinweises auf die Patenterteilung: **25.02.2009**
(21) Anmeldenummer: 06721203.5
(22) Anmeldetag: 11.04.2006
(51) Int. Cl.: A61F 5/00

(54) **Steuerbares Magenband**
Adjustable stomach band
Anneau gastrique réglable

(30) Priorität: 11.04.2005 AT 6012005
(43) Veröffentlichungstag der Anmeldung: 26.12.2007
(73) Patentinhaber: Lechner, Wolfgang, 3441 Judenau/Pixendorf (AT)
(72) Erfinder: Lechner, Wolfgang, 3441 Judenau/Pixendorf (AT)
(74) Vertreter: Sonn & Partner Patentanwälte
(86) Internationale Anmeldenummer: PCT/AT2006/000145
(87) Internationale Veröffentlichungsnummer: WO 2006/108203

(56) Entgegenhaltungen:
- EP-A- 0 876 808
- WO-A-20/04014245
- WO-A-20/05009305
- US-A- 4 721 509
- US-B1- 6 475 136

## Beschreibung

Die Erfindung betrifft ein steuerbares Magenband mit einem nicht dehnbaren Rücken und einer Stoma-seitig vom Rücken angeordneten Kammer zur Steuerung der Stomaeinengung durch Zu- bzw. Abfuhr von Flüssigkeit bzw. einem Fluid in die bzw. aus der Kammer, wobei außerhalb des Rückens eine Druckkammer vorgesehen ist, welche über ein Druckventil mit der Stoma-einengenden Kammer verbunden ist.

Die Erfindung bezieht sich auf eine Weiterentwicklung des steuerbaren Magenbandes, wie es von mehreren Firmen in prinzipiell gleicher Bauform angeboten wird (z.B. Schwedenband der Fa. Obtech (Johnson & Johnson), Lapband der Fa. Bioenterics,...). Es handelt sich hierbei um ein zur Restriktion der Nahrungsaufnahme eingesetztes Band, das um den obersten Magenteil bzw. Ösophagus herum geschlungen und verschlossen wird.

Die WO 01/24742 A1 beschreibt ein Magenband, welches gürtelförmig um den Magen herumgelegt und befestigt wird. Eine Einstellung der Einengung des Stomas ist rein mechanisch durch Einengung des Bandes möglich.

Die US 4 592 339 A beschreibt ein Magenband, bei dem an der dem Magen zugewandten Seite des Bandes eine Kammer angeordnet ist, die mit Flüssigkeit aufgefüllt werden kann. Dadurch ist die Steuerung der Stomaweite möglich. Über einen subkutan eingenähten Port, der über einen Schlauch mit der Kammer des Magenbandes verbunden ist, kann eine Flüssigkeitsfüllung und Entleerung des Systems durchgeführt werden.

Die WO 03/020183 A1 zeigt ein Magenband, welches zur Schonung des Magens mit einem viskoelastischen Material umgeben ist.

Schließlich zeigt die WO 2005/009305 A1 ein Magenband, welches eine mechanisch bzw. elektrisch gesteuerte und bewirkte autoregulatorische Änderung der Stomaweite aufweist, um Probleme, die mit dem derzeit verwendeten Magenband auftreten, zu umgehen und bessere Langzeitergebnisse zu erreichen. Denn die derzeit verwendeten Magenbänder bringen zwar in der Mehrzahl der Fälle gute Langzeitergebnisse hinsichtlich Gewichtsreduktion und Patientenzufriedenheit, aber es gibt einige Probleme die besonders bei hoher Bandauffüllung in den Vordergrund treten. Viele Patienten berichten dann über die unangenehme Erscheinung des Speichel-Erbrechens bzw. Herauswürgens, v.a. beim flachen Liegen. Speisereste können lange oberhalb des Stomas in der Speiseröhre verbleiben, hier zu gären beginnen und dadurch neben einem unangenehmen Mundgeruch eine Schleimhautreizung mit entsprechenden Schmerzen hervorrufen. Die ununterbrochen bestehende hohe Engstellung des Stomas führt im Verlauf von Monaten zu einer Schwächung der Ösophagusmotilität bzw. in einigen Fällen zu einer zunehmenden Ausdehnung der Speiseröhre, wodurch schließlich die Ösophagus-Sensibilität schwindet und die Bandwirkung verloren geht, was dann zu einer Gewichtszunahme trotz liegendem hoch aufgefülltem Magenband führt.

Die WO 2005/009305 A1 versucht das mit den derzeit verwendeten Magenbändern gegebene Problem dadurch auszuschalten, dass die eingestellte Stomaweite nicht ständig gleich bleibt, sondern sich in Abhängigkeit vom Bedarf autoregulatorisch verändert. Angestrebt wird hier eine zunehmende Stomaeinengung während des Essens, welche sich nach Abschluss der Nahrungsaufnahme wieder rückbildet.

Ausgehend vom Stand der Technik beim derzeit verwendeten Magenband und im Unterschied zur eben genannten Patentanmeldung WO 2005/009305 A1 zielt die Erfindung darauf ab, ein Magenband zu schaffen, bei dem bei entsprechendem Druckanstieg in der Kammer des Magenbandes ein vorübergehendes Weiterwerden des Stomas und damit ein Passieren des Bolus durch das Stoma ermöglicht wird. Ziel ist die Schaffung eines Magenbandes mit dynamischer Veränderung der Stomaweite.

Gelöst wird die erfindungsgemäße Aufgabe durch ein oben genanntes Magenband, bei dem das Druckventil in seinem Öffnungsverhalten steuerbar ausgebildet ist. Das Magenband wird in seiner Stomaweite so eng eingestellt, dass es für aufgenommene Nahrungsmittel kaum noch durchgängig ist. Die Ösophagusperistaltik befördert den Bolus in den kleinen Magenteil oberhalb des Bandes. Das enge Band bewirkt eine Abflussbehinderung für den Bolus. Dadurch entsteht ein hoher Intrabolusdruck der schließlich den Druck der peristaltischen Welle erreicht (40-80 mmHg). Das erfindungsgemäße Druckventil wird beim Druckanstieg durch den Bolus geöffnet, ein Teil der Flüssigkeit aus der Stoma-einengenden Kammer des Magenbandes tritt in die Druckkammer über, wodurch das Stoma weiter wird und der Bolus passieren kann. Mit dem erfindungsgemäßen Magenband wird ein Verzögerungseffekt auf die Nahrungspassage bewirkt und für den Bandträger ein frühes Völlegefühl erreicht, und somit die aufgenommene Nahrungsmenge reduziert. Durch entsprechende Adjustierung des Druckventils kann das Verhalten des Magenbandes verändert und an die individuelle Situation beim Patienten angepasst werden. Bei den derzeit zur Verfügung stehenden Magenbändern bleibt die eingestellte Stomaweite üblicherweise starr.

Gemäß einem weiteren Merkmal der Erfindung ist das Druckventil hinsichtlich der Druckhöhe, bei welcher das Druckventil öffnet, steuerbar ausgebildet.

Die Steuerung des Druckventils kann beispielsweise mechanisch durchgeführt werden.

Dabei ist es möglich, die mechanische Steuerung des Druckventils mittels Flüssigkeit bzw. einem Fluid vorzunehmen.

Ebenso kann die mechanische Steuerung des Druckventils über eine zweite Portkammer erfolgen, welche mittels eines Verbindungsschlauchs mit dem Druckventil verbunden ist.

Schließlich kann die Steuerung des Druckventils auch elektronisch erfolgen.

Vorteilhafterweise ist die Druckkammer elastisch ausgebildet, so dass diese aufgrund ihrer elastischen Eigenschaften befähigt ist, Volumen unter einem erhöhten Druck zu speichern. Somit wird die bei Öffnung des Druckventils in den Druckbehälter abfließende Flüssigkeit bzw. das Fluid unter erhöhtem Druck in der Druckkammer zwischengespeichert.

Um zu ermöglichen, dass nach der Passage des Bolus wieder ein erhöhter Druck durch die Stoma-einengende Kammer auf den Magen ausgeübt wird, ist die Druckkammer über einen Rückflusskanal mit Rückschlagventil mit der Stoma-einengenden Kammer verbunden. Somit entleert sich die in der Druckkammer zwischengespeicherte Flüssigkeit bzw. das Fluid über den Rückflusskanal und das Rückschlagventil unmittelbar nach der Passage des Bolus wieder in die Stoma-einengende Kammer des Magenbandes, und der Ausgangszustand ist wieder hergestellt.

Vorteilhafterweise ist das Druckventil mit einer Einrichtung zur zeitlichen Steuerung verbunden. Dadurch ist es möglich, das Verhalten des Druckventils in Abhängigkeit der Zeit zu beeinflussen. Beispielsweise kann das Druckventil während der Nachtstunden so eingestellt werden, dass bereits bei einem geringen Druckanstieg die Stoma-einengende Kammer weiter wird und somit die Passage eines Bolus oder das Abfließen von Speichel ermöglicht. Demgegenüber kann der Druck, bei welchem das Druckventil öffnet, in den Morgenstunden, höher eingestellt werden, so dass die Nahrungsaufnahme in der Früh erschwert wird.

Wenn eine mit dem Druckventil verbundene Einrichtung zum Detektieren von peristaltischen Wellen vorgesehen ist, kann das Verhalten des Druckventils auch in Abhängigkeit der Nahrungsaufnahme beeinflusst und gesteuert werden. Beispielsweise kann zu Beginn eines Essvorgangs, d.h. bei den ersten durchlaufenden peristaltischen Wellen und damit Druckanstiegen das Druckventil bereits bei geringen Steigerungen des Druckes, beispielsweise von 30 mmHg, vollständig geöffnet werden, wodurch der Bolus problemlos passieren kann. Nach einigen Minuten werden höhere Öffnungsdrucke erforderlich und bzw. oder nur jeder zweite oder dritte Druckanstieg führt zu einer Öffnung des Druckventils. Der dadurch erzeugte Rückstau des Bolus bewirkt ein zunehmendes Völlegefühl. Erbrechen wird verhindert, indem die entstehenden hohen Drucke eine Öffnung des Druckventils bewirken, wodurch sich das Stoma erweitert und der Bolus passieren kann. Einige Minuten nach Beendigung der Mahlzeit nimmt das Druckventil wieder das Ausgangsverhalten an und öffnet bereits bei geringen Druckanstiegen. Die bei unvollständigem Abfluss des Bolus ausgelösten sekundären peristaltischen Wellen können die Speisereste zum Abfließen bringen.

Wenn Stoma-seitig vom Rücken zumindest eine Hilfskammer vorgesehen ist, kann eine Anpassung des Magenbandes an die Dicke der Magenwand sowie die im Magenband zusätzlich eingeschlossene Menge an Fett und Bindegewebe, welche von Mensch zu Mensch verschieden ist, erfolgen. Durch Befüllung der zumindest einen Hilfskammer, kann der Basisdruck im Magenband eingestellt werden. Mit zunehmendem Füllvolumen der Hilfskammer steigt der Druck auch in der Stoma-einengenden Kammer an. Diese zumindest eine Hilfskammer ist in den Flüssigkeitskreislauf zwischen der Stoma-einengenden Kammer und der Druckkammer vorzugsweise nicht eingebunden.

Zur Einstellung des Basisdrucks ist die Stoma-einengende Kammer und bzw. oder die zumindest eine Hilfskammer vorzugsweise mit einem subkutan anzuordnenden Port verbunden. Über Befüllung des Ports mit Flüssigkeit oder einem Fluid bzw. Absaugen von Flüssigkeit aus dem Port kann der Basisdruck eingestellt werden. Selbstverständlich sind auch autonom arbeitende Ports, bei welchen die Flüssigkeit bzw. das Fluid von einem Reservoir in die Stoma-einengende Kammer bzw. die zumindest eine Hilfskammer verschoben wird, möglich.

Gemäß einer weiteren Ausführungsform der Erfindung ist die Druckkammer im oder neben dem Port angeordnet. Das bedeutet, dass die Druckkammer, welche über das Druckventil mit der Stoma-einengenden Kammer verbunden ist, nicht unbedingt in der Nähe des Rückens des Magenbandes angeordnet sein muss, sondern auch beispielsweise im oder neben dem subkutan angeordneten Port. Wenn das Druckventil bei geringstem Druck öffnet, hat das Magenband die Eigenschaft Druck-stabilisierend zu wirken. Der Anstieg des Drucks in der Stoma-einengenden Kammer wird in der Druckkammer aufgefangen und damit abgeschwächt. Die Charakteristik und somit die Eigenschaften des Magenbandes hängen von den elastischen Eigenschaften der Druckkammer ab.

Die Erfindung wird anhand der beigefügten Abbildungen näher erläutert.

Darin zeigen:
- Fig. 1: einen schematischen Querschnitt durch eine Ausführungsform

- Fig. 2: eines Magenbandes vor Öffnung des Druckventils; einen schematischen Querschnitt durch das Magenband gemäß Fig. 1 nach Flüssigkeitsverlagerung in die Druckkammer;
- Fig. 3: eine weitere Ausführungsform eines Magenbandes mit subkutan angeordnetem Port;
- Fig. 4: eine weitere Ausführungsform eines Magenbandes mit subkutanem Port und daneben angeordneter Druckkammer;
- Fig. 5: eine weitere Ausführungsform eines Magenbandes mit zumindest einer Hilfskammer; und
- Fig. 6: eine schematische Darstellung eines implantierten Magenbandes mit subkutan angeordnetem Port und Sensoren zur Detektion peristaltischer Wellen sowie der Schluckaktivität des Patienten.

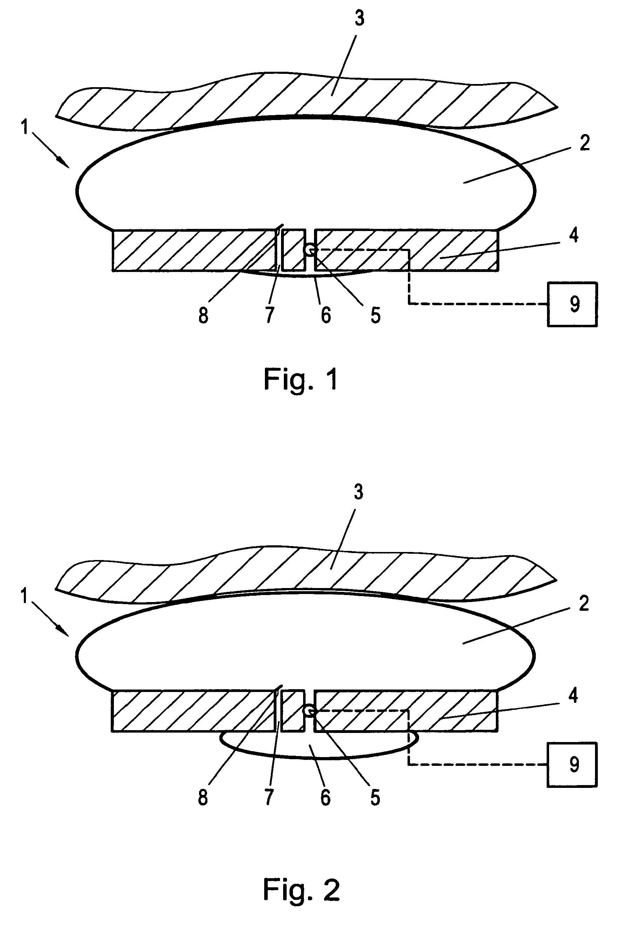

Fig. 1 zeigt einen Querschnitt durch ein Magenband 1 mit einer Flüssigkeits-gefüllten Kammer 2 und einem nicht dehnbaren Rücken 4. Die Flüssigkeits-gefüllte Kammer 2 liegt an der Magenwand 3 an, so dass der Magen in Abhängigkeit der Füllung der Kammer 2 mehr oder weniger eingeengt werden kann. Erfindungsgemäß ist die Stoma-einengende Kammer 2 über ein Druckventil 5 mit einer außerhalb des Rückens 4 angeordneten Druckkammer 6 verbunden. Über einen Rückflusskanal 7 mit einem Rückschlagventil 8 kann die Flüssigkeit aus der Druckkammer 6 wieder in die Stoma-einengende Kammer 2 zurückgeleitet werden. Anstelle einer Flüssigkeit kann theoretisch auch ein Gas als Füllung für die Kammer 2 verwendet werden.

Bei Druckanstieg in der Kammer 2 öffnet das Druckventil 5 und Flüssigkeit aus der Kammer 2 tritt in die Druckkammer 6 über. Die Druckkammer 6 kann elastisch ausgeführt sein und hat dadurch die Eigenschaft, die eingepresste Flüssigkeit unter erhöhtem Druck zu speichern. Damit wird das Stoma weiter, ein Bolus kann leichter passieren. Diese Situation ist in Fig. 2 dargestellt. Nach der Passage eines Bolus fällt der Druck in der Kammer 2 wieder ab, so dass die in der Druckkammer 6 unter erhöhtem Druck zwischengespeicherte Flüssigkeit über den Rückflusskanal 7 mit dem Rückschlagventil 8 wieder in die Kammer 2 zurückfließen kann.

Das Druckventil 5 ist in seinem Öffnungsverhalten vorzugsweise steuerbar ausgebildet, wobei diese Steuerung mechanisch oder elektronisch erfolgen kann. Weiters kann das Druckventil 5 mit einer Einrichtung 9 zur zeitlichen Steuerung verbunden sein, wie in den Fig. 1 und 2 angedeutet. Dadurch kann das Druckventil 5 von der Tageszeit abhängig gesteuert werden. Beispielsweise kann der Druck, bei welchem das Druckventil 5 öffnet, während der Morgenstunden höher eingestellt werden, so dass die Nahrungsaufnahme in den Morgenstunden erschwert wird. Ebenso kann der Druck, bei welchem das Druckventil 5 öffnet, während der Nachtstunden reduziert werden, um zu erreichen, dass Speichel oder oberhalb des Stomas gestaute Speisereste das Stoma passieren und abgeführt werden können.

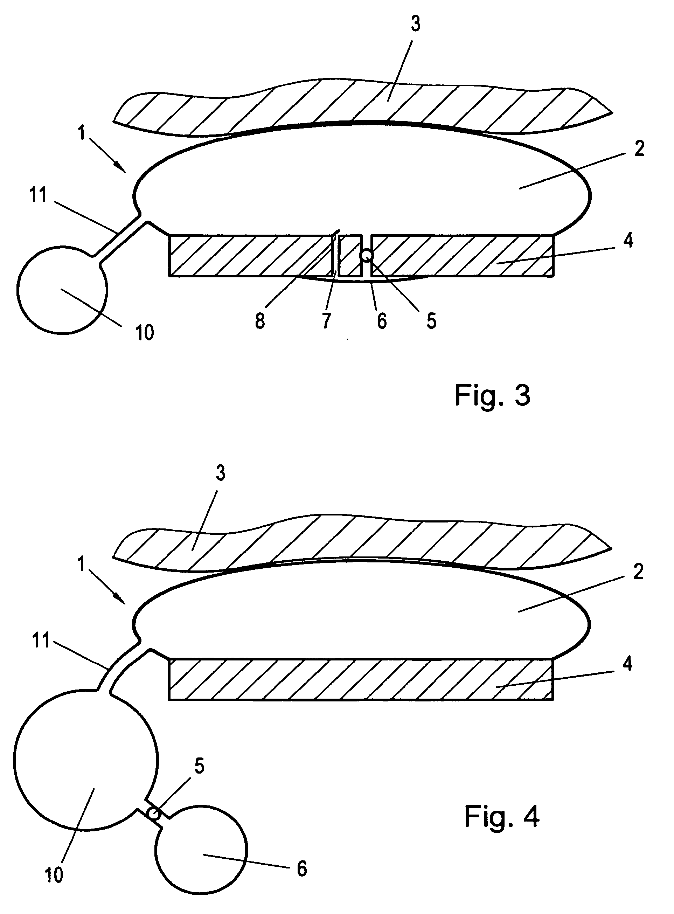

Fig. 3 zeigt eine Variante des erfindungsgemäßen Magenbandes 1, wobei die Stoma-einengende Kammer 2 über eine entsprechende Leitung 11 mit einem subkutan anzuordnenden Port 10 verbunden ist. Über Zuführung bzw. Abführung von Flüssigkeit über den Port 10 in bzw. aus der Stoma-einengenden Kammer 2, kann eine Anpassung des Magenbandes 1 an die jeweiligen Gegebenheiten vorgenommen werden.

Bei der Ausführungsvariante eines Magenbandes 1 gemäß Fig. 4 ist die Druckkammer 6 nicht unmittelbar hinter dem Rücken 4 sondern neben dem Port 10 angeordnet. Bei einem Anstieg des Drucks in der Stoma-einengenden Kammer 2 wird dieser über die Leitung 11 in den Port 10 weitergeleitet, wo bei Erreichen eines entsprechenden Drucks das Druckventil 5 öffnet und die Flüssigkeit in die Druckkammer 6 geleitet wird. Bei Reduktion des Drucks in der Stoma-einengenden Kammer 2 wird die Flüssigkeit wieder aus der Druckkammer 6 in den Port 10 geleitet. Dies kann beispielsweise durch ein spezielles Druckventil 5, welches in beide Richtungen funktioniert oder über einen Rückflusskanal, wie er in den Ausführungsformen gemäß den Fig. 1 bis 3 dargestellt wurde, erreicht werden.

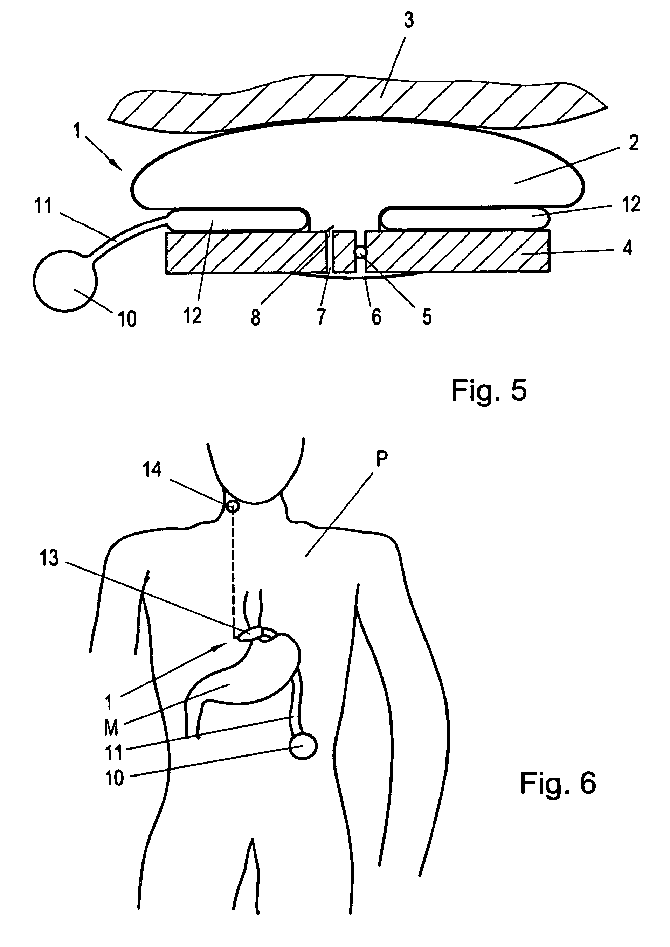

Die Ausführungsform eines Magenbandes 1 gemäß Fig. 5 unterscheidet sich gegenüber der Variante gemäß Fig. 1 dadurch, dass unterhalb der Stoma-einengenden Kammer 2 zumindest eine Hilfskammer 12 beispielsweise ringförmig angeordnet ist, welche über eine Leitung 11 mit einem subkutan anzuordnenden Port 10 verbunden ist. Über diese Hilfskammer 12 kann die Grundeinstellung des Drucks des Magenbandes 1 erfolgen. Die Hilfskammern 12 sind dabei nicht in den Flüssigkeitskreislauf zwischen der Stoma-einengenden Kammer 2 und der Druckkammer 6 eingebunden. Durch Zuführung bzw. Abführung von Flüssigkeit in den bzw. aus dem Port 10 kann eine Anpassung des Magenbandes 1 an die individuell unterschiedlichen Schichtdicken der Magenwand 3 und durch das Magenband 1 eingeschlossenes Fettgewebe erreicht werden.

Fig. 6 zeigt schematisch eine Anwendung des erfindungsgemäßen Magenbandes 1, welches den Eingang des Magens M des Patienten P umschließt. Über eine Leitung 11 ist die Stoma-einengende Kammer 2 (nicht dargestellt) des Magenbandes 1 mit einem subkutan anzuordnenden Port 10 verbunden, über welchen der Basisdruck, welcher das Magenband 1 auf die Magenwand ausübt, eingestellt werden kann. Das Druckventil 5 des Magenbandes 1 kann beispielsweise mit einer Einrichtung 13 zum Detektieren von peristaltischen Wellen verbunden sein, so dass in Abhängigkeit der peristaltischen Wellen bzw. der Essensaufnahme eine Steuerung des Druckventils 5 möglich wird. Ebenso kann das Druckventil 5 auch mit einem Sensor 14 zur Messung der Schluckaktivität verbunden sein. Die Verbindung zwischen dem Sensor 14 zur Messung der Schluckaktivität mit dem Druckventil 5 bzw. einer entsprechenden Elektronik (nicht dargestellt) kann beispielsweise über Funk erfolgen.

## Patentansprüche

1. Steuerbares Magenband (1) mit einem nicht dehnbaren Rücken (4) und einer Stoma-seitig vom Rücken (4) angeordneten Kammer (2) zur Steuerung der Stomaeinengung durch Zu- bzw. Abfuhr von Flüssigkeit bzw. einem Fluid in die bzw. aus der Kammer (2), wobei außerhalb des Rückens (4) eine Druckkammer (6) vorgesehen ist, welche über ein Druckventil (5) mit der Stoma-einengenden Kammer (2) verbunden ist, **dadurch gekennzeichnet, dass** das Druckventil (5) in seinem Öffnungsverhalten steuerbar ausgebildet ist.

2. Magenband (1) nach Anspruch 1, **dadurch gekennzeichnet, dass** das Druckventil (5) hinsichtlich der Druckhöhe, bei welcher das Druckventil (5) öffnet, steuerbar ausgebildet ist.

3. Magenband (1) nach Anspruch 1 oder 2, **dadurch gekennzeichnet, dass** die Steuerung des Druckventils (5) mechanisch erfolgt.

4. Magenband (1) nach Anspruch 3, **dadurch gekennzeichnet, dass** die mechanische Steuerung des Druckventils (5) mittels Flüssigkeit bzw. einem Fluid erfolgt.

5. Magenband (1) nach Anspruch 3 oder 4, **dadurch gekennzeichnet, dass** die mechanische Steuerung des Druckventils (5) über eine zweite Portkammer erfolgt, welche mittels eines Verbindungsschlauchs mit dem Druckventil (5) verbunden ist.

6. Magenband (1) nach Anspruch 1 oder 2, **dadurch gekennzeichnet, dass** die Steuerung des Druckventils (5) elektronisch erfolgt.

7. Magenband (1) nach einem der Ansprüche 1 bis 6, **dadurch gekennzeichnet, dass** die Druckkammer (6) elastisch ausgebildet ist, so dass diese aufgrund ihrer elastischen Eigenschaften befähigt ist, Volumen unter einem erhöhten Druck zu speichern.

8. Magenband (1) nach einem der Ansprüche 1 bis 7, **dadurch gekennzeichnet, dass** die Druckkammer (6) über einen Rückflusskanal (7) mit Rückschlagventil (8) mit der Stoma-einengenden Kammer (2) verbunden ist, so dass die in der Druckkammer (6) unter erhöhtem Druck gespeicherte Flüssigkeit wieder in die Stoma-einengende Kammer (2) zurückgeleitet werden kann.

9. Magenband (1) nach einem der Ansprüche 1 bis 8, **dadurch gekennzeichnet, dass** das Druckventil (5) mit einer Einrichtung (9) zur zeitlichen Steuerung verbunden ist.

10. Magenband (1) nach einem der Ansprüche 1 bis 9, **dadurch gekennzeichnet, dass** eine mit dem Druckventil (5) verbundene Einrichtung (13) zum Detektieren von peristaltischen Wellen vorgesehen ist.

11. Magenband (1) nach einem der Ansprüche 1 bis 10, **dadurch gekennzeichnet, dass** Stoma-seitig vom Rücken (4) zumindest eine Hilfskammer (12) vorgesehen ist.

12. Magenband (1) nach einem der Ansprüche 1 bis 11, **dadurch gekennzeichnet, dass** die Stoma-einengende Kammer (2) und bzw. oder die zumindest eine Hilfskammer (12) mit einem subkutan anzuordnenden Port (10) verbunden ist.

13. Magenband (1) nach Anspruch 12, **dadurch gekennzeichnet, dass** die Druckkammer (6) im oder neben dem Port (10) angeordnet ist.

14. Magenband (1) nach einem der Ansprüche 1 bis 13, **dadurch gekennzeichnet, dass** ein Sensor (14) zur Messung der Schluckaktivität vorgesehen ist, welcher mit dem Druckventil (5) verbunden ist.

## Claims

1. A controllable gastric band (1) including a nonextensible back (4) and a chamber (2) arranged on the stoma side of the back (4) for controlling the stoma restriction by supplying and discharging a liquid, or a fluid, respectively, to and from said chamber (2), a pressure chamber (6) being provided on the exterior of the back (4), which pressure chamber is connected to the stoma-restricting chamber (2) via a pressure valve (5), **characterised in that** the pressure valve (5) is designed to be controllable in its opening behaviour.

2. A gastric band (1) according to claim 1, **characterised in that** the pressure valve (5) is designed to be controllable with respect to the pressure level at which the pressure valve (5) will open.

3. A gastric band (1) according to claim 1 or 2, **characterised in that** the control of the pressure valve (5) is effected mechanically.

4. A gastric band (1) according to claim 3, **characterised in that** the mechanical control of the pressure valve (5) is effected by the aid of a liquid, or a fluid, respectively.

5. A gastric band (1) according to claim 3 or 4, **characterised in that** the mechanical control of the pressure valve (5) is effected via a second port chamber, which is connected with the valve (5) by a connection hose.

6. A gastric band (1) according to claim 1 or 2, **characterised in that** the control of the pressure valve (5) is effected electronically.

7. A gastric band (1) according to any one of claims 1 to 6, **characterised in that** the pressure chamber (6) is designed to be elastic so that, due to its elastic properties, it is capable of storing volume at an elevated pressure.

8. A gastric band (1) according to any one of claims 1 to 7, **characterised in that** the pressure chamber (6) is connected to the stoma-restricting chamber (2) via a backflow channel (7) including a non-return valve (8) so that the liquid stored in the pressure chamber (6) at an elevated pressure can be conveyed back into the stoma-restricting chamber (2).

9. A gastric band (1) according to any one of claims 1 to 8, **characterised in that** the pressure valve (5) is connected to a means (9) for a temporal control.

10. The gastric band (1) according to any one of claims 1 to 9, **characterised in that** a means (13) for detecting peristaltic waves is provided, which means (13) is connected to the pressure valve (5).

11. A gastric band (1) according to any one of claims 1 to 10, **characterised in that** on the stoma-side of the back (4), at least one auxiliary chamber (12) is provided.

12. A gastric band (1) according to any one of claims 1 to 11, **characterised in that** the stoma-restricting chamber (2) and/or the at least one auxiliary chamber (12) are connected to a port (10) to be arranged subcutaneously.

13. A gastric band (1) according to claim 12, **characterised in that** the pressure chamber (6) is arranged in or adjacent to the port (10).

14. A gastric band (1) according to any one of claims 1 to 13, **characterised in that** a sensor (14) is provided for measuring the swallowing activity, which sensor is connected to the pressure valve (5).

## Revendications

1. Bande gastrique (1) ajustable de manière commandée, comprenant un dos (4) non extensible et une chambre (2) agencée côté stomacal du dos (4) pour commander le rétrécissement stomacal par l'amenée ou l'évacuation de liquide ou d'un fluide dans la chambre (2) et respectivement hors de la chambre (2), une chambre de pression (6) étant prévue à l'extérieur du dos (4) et étant reliée à la chambre (2) de rétrécissement stomacal par l'intermédiaire d'une soupape de pression (5), **caractérisée en ce que** la soupape de pression (5) est conçue pour permettre d'être commandée quant à son comportement à l'ouverture.

2. Bande gastrique (1) selon la revendication 1, **caractérisée en ce que** la soupape de pression (5) est d'une configuration permettant la commande quant au niveau de pression pour lequel la soupape de pression (5) ouvre.

3. Bande gastrique (1) selon la revendication 1 ou 2, **caractérisée en ce que** la commande de la soupape de pression (5) s'effectue mécaniquement.

4. Bande gastrique (1) selon la revendication 3, **caractérisée en ce que** la commande mécanique de la soupape de pression (5) s'effectue au moyen de liquide ou d'un fluide.

5. Bande gastrique (1) selon la revendication 3 ou 4, **caractérisée en ce que** la commande mécanique de la soupape de pression (5) s'effectue par l'intermédiaire d'une deuxième chambre de port de branchement, qui est reliée à la soupape de pression (5) au moyen d'une tubulure de liaison.

6. Bande gastrique (1) selon la revendication 1 ou 2, **caractérisée en ce que** la commande de la soupape de pression (5) s'effectue par voie électronique.

7. Bande gastrique (1) selon l'une des revendications 1 à 6, **caractérisée en ce que** la chambre de pression (6) est de configuration élastique, de sorte qu'elle est en mesure, en raison de ses propriétés élastiques, d'accumuler ou de stocker des volumes sous une pression plus élevée.

8. Bande gastrique (1) selon l'une des revendications 1 à 7, **caractérisée en ce que** la chambre de pression (6) est reliée à la chambre (2) de rétrécissement stomacal par l'intermédiaire d'un canal d'écoulement de retour (7) avec un clapet anti-retour (8), de sorte que le liquide accumulé ou stocké sous une pression plus élevée dans la chambre de pression (6), peut à nouveau être renvoyé dans la chambre (2) de rétrécissement stomacal.

9. Bande gastrique (1) selon l'une des revendications 1 à 8, **caractérisée en ce que** la soupape de pression (5) est reliée à un dispositif (9) pour une commande en fonction du temps.

10. Bande gastrique (1) selon l'une des revendications 1 à 9, **caractérisée en ce qu'**il est prévu un dispositif (13) relié à la soupape de pression (5) et destiné à détecter des ondes péristaltiques.

11. Bande gastrique (1) selon l'une des revendications 1 à 10, **caractérisée en ce qu'**il est prévu, côté estomac du dos (4), au moins une chambre auxiliaire (12).

12. Bande gastrique (1) selon l'une des revendications 1 à 11, **caractérisée en ce que** la chambre (2) de rétrécissement stomacal et respectivement ou ladite une chambre auxiliaire (12) est ou sont reliées à un port de branchement (10) à implanter en position sous-cutanée.

13. Bande gastrique (1) selon la revendication 12, **caractérisée en ce que** la chambre de pression (6) est agencée dans ou à côté du port de branchement (10).

14. Bande gastrique (1) selon l'une des revendications 1 à 13, **caractérisée en ce qu'**il est prévu un détecteur (14) pour mesurer l'activité de déglutition, qui est relié à la soupape de pression (5).
